(19) Europäisches Patentamt
European Patent Office
Office européen des brevets

(11) Veröffentlichungsnummer: **0 010 208**
**B1**

(12) # EUROPÄISCHE PATENTSCHRIFT

(45) Veröffentlichungstag der Patentschrift: **11.08.82**

(51) Int. Cl.³: **A 61 K 31/20**, A 61 K 31/07, C 07 C 175/00

(21) Anmeldenummer: **79103681.7**

(22) Anmeldetag: **28.09.79**
**Die Akte enthält technische Angaben, die nach dem Eingang der Anmeldung eingereicht wurden und die nicht in dieser Patentschrift enthalten sind.**

(54) **Pharmazeutisches Mittel, enthaltend all-E- oder 13-Z-7,8-Dehydro-Retinsäure und Verfahren zu seiner Herstellung.**

(30) Priorität: **07.10.78 DE 2843901**

(43) Veröffentlichungstag der Anmeldung: **30.04.80 (Patentblatt 80/09)**

(45) Bekanntmachung des Hinweises auf die Patenterteilung: **11.08.82 Patentblatt 82/32**

(84) Benannte Vertragsstaaten: **AT BE CH DE FR GB IT LU NL SE**

(56) Entgegenhaltungen:
**FR A 2 081 478**
**FR A 2 100 815**
**GB A 722 911**

**JOURNAL OF THE CHEMICAL SOCIETY, 1952 (I) London, GB, J. AZZENBURROW: « A synthesis of vitamin A from cyclohexanone », Seiten 1 094-111**

(73) Patentinhaber: **BASF Aktiengesellschaft**
**Carl-Bosch-Strasse 38**
**D-6700 Ludwigshafen (DE)**

(72) Erfinder: **Paust, Joachim, Dr.**
**Ringstrasse 3**
**D-6701 Neuhofen (DE)**
Erfinder: **Hoffmann, Werner, Dr.**
**Ringstrasse 11c**
**D-6701 Neuhofen (DE)**
Erfinder: **Baumann, Manfred, Dr.**
**Im Sennteich 28**
**D-6800 Mannheim 24 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 208 B1

Imprimerie Jouve, 18, rue St-Denis, 75001 Paris, France

# Pharmazeutisches Mittel, enthaltend all-E- oder 13-Z-7,8-Dehydro-Retinsäure und Verfahren zu seiner Herstellung

Die Erfindung betrifft ein pharmazeutisches Mittel, gekennzeichnet durch einen Gehalt an all-E- oder 13-Z-7,8-Dehydroretinsäure als Wirkstoff neben üblichen pharmazeutischen Trägerstoffen und Verdünnungsmitteln und die Herstellung dieser Mittel.

Es ist bekannt, daß Vitamin-A-Verbindungen z.B. Ester des Retinols oder die Retinsäure, die normale Zelldifferenzierung von epithelialem Gewebe kontrollieren. Die Wirksamkeit von Retinol und Retinsäure bei der Verhütung von Lugen-, Haut-, Blasen- und Brustkrebs im Tierversuch ist ebenfalls von mehreren Arbeitskreisen beschrieben (W. Bollag, Int. Z. Vitaminforsch. *40,* 299 ff (1970); M. B. Sporn et al., Federation Proc. Fed. Am. Soc. Exp. Biol. *35,* 1332-1338 (1976)). Die natürlichen Vitamin-A-Verbindungen zeigen jedoch neben ihrer krebshemmenden Wirksamkeit in Tierversuchen und beim Menschen als Nachteile eine relativ hohe Toxizität, bedingt durch eine ungünstige Verteilung im Organismus (Pharmakokinetik). Beispielsweise wird Retinol vorwiegend in Form des Retinylpalmitats in der Leber gespeichert, wo es in höherer Konzentration ernsthafte Funktionsstörungen bewirkt. Höhere Dosen an Retinsäure führen zu Anzeichen an schwerer systemischer Toxizität, die durch Destabilisierung von Membranen, Bruch von Lysosomen, etc. bis hin zu Knochenbrüchen charakteriziert sein kann.

Es wurde nun gefunden, daß diese Nachteile vermieden werden können durch ein pharmazeutisches Mittel, gekennzeichnet durch einen Gehalt an all-E- oder 13-Z-7,8-Dehydro-retinsäure als Wirkstoff neben üblichen pharmazeutischen Trägerstoffen oder Verdünnungsmitteln und gegebenenfalls üblichen pharmazeutisch-technischen Hilfsstoffen.

Die all-E- und 13-Z-7,8-Dehydro-retinsäure ähneln in ihrer krebsprophylaktischen Wirkung der Retinsäure und sind beispielsweise am durch Vitamine A-Hypovitaminose keratinisierten Hamster-Tracheen-Gewebe noch in $10^{-9}$ molarer Konzentration wirksam. Die Methodik hierzu kann G. H. Clamon et al., Nature *250,* 64-66 (1974) oder M. B. Sporn et al., Nature *253,* 47-50 (1975) entnommen werden. Die Keratinisierung wird als praekanzerotischer Prozeß angesehen. Im Vergleich zur Retinsäure zeigen sie jedoch überraschenderweise eine geringere Toxizität und günstigere Pharmakokinetik. Die für eine Therapie oder Prophylaxe von Praekanzerosen bzw. Karzinomen erforderliche Wirkstoffkonzentration kann daher in den betreffenden Organen ohne unerwünschte Nebenwirkungen erreicht werden.

Die tumorhemmende Wirkung der erfindungsgemäßen Verbindungen ist signifikant. Man beobachtet eine Wachstumskontrolle bei in vitro kultivierten Zellen, die beispielsweise nach der Methodik, wie sie von R. Lotan et al., im Journal of the National Cancer Institute *60* (1978) Seiten 1035-1041, beschrieben wird, nachgewiesen werden kann. Die Erfindungsgemäßen Verbindungen inhibieren die Proliferation von spontan chemisch oder durch Viren transformierter Zellen in Gewebekultur, vorzugsweise verwendet man die S 91 Melanoma Zellinie.

Dementsprechend kann das erfindungsgemäße Mittel zur topischen und systemischen Therapie und Prophylaxe von Praekanzerosen und Karzinomen der Haut, Schleimhäute und innerer Organe sowie bei der topischen und systemischen Therapie von Akne, Psoriasis und anderen mit pathologisch veränderter Verhornung einhergehenden dermatologischen Erkrankungen verwendet werden.

Ein bevorzugtes Indikationsgebiet ist die prophylaktische und therapeutische Behandlung von Praekanzerosen und Tumoren der Blase, der Brustdrüse, der Haut und der Schleimhäute.

Die 13-Z-7,8-Dehydro-retinsäure (I) kann in einfacher Weise ausgehend von 7,8-Dehydro-β-ionyliden-acetaldehyd III über 7,8-Dehydro-β-ionyliden-ethyl-triphenyl-phosphonium IV durch Wittig-Olefinierung mit dem Lactol der Z-3-formyl-2-butensäure V hergestellt werden.

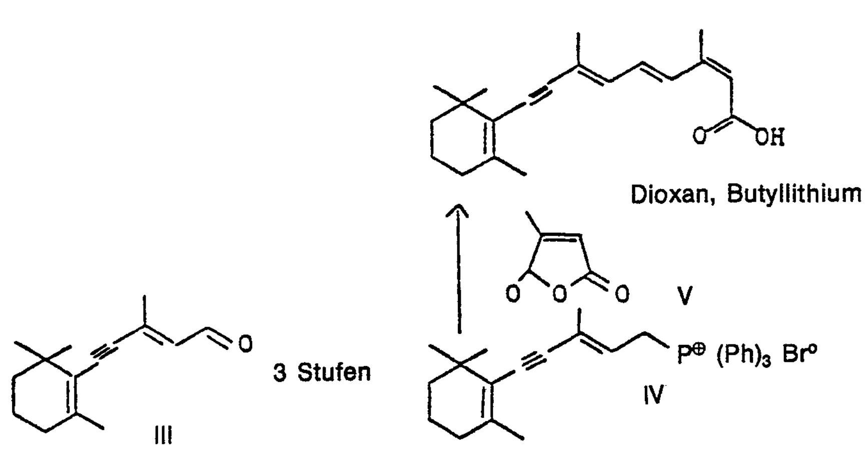


I

V

IV

III

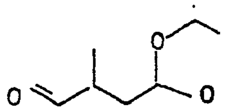


VIII

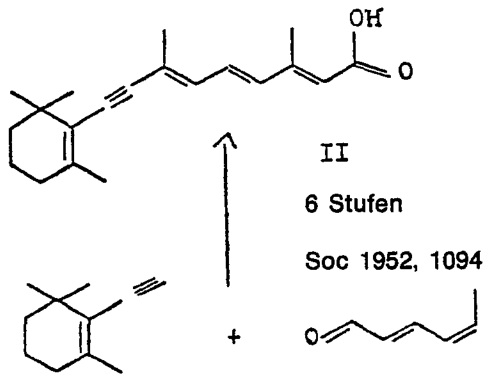


II

VI VII

Eine Synthese der all-E-7,8-Dehydro-retinsäure (II) in sechs Stufen aus 2-Ethinyl-1,3,3-trimethylcyclohex-1-en VI und Hepta-3,5-dien-2-on VII ist in J. Chem. Soc. (London), *1952*, 1094 ff (J. Attenburrow et al.) beschrieben.

Ein besserer Zugang zu II eröffnet sich ausgehend von IV durch Wittig-Olefinierung mit E-3-Formyl-2-butensäure-ethyl-ester VIII und Hydrolyse der Esterfunktion.

Es sei hervorgehoben, daß sich die all-E- und 13-Z-7,8-Dehydro-retinsäure gegenüber Retinol und Retinsäure auch durch eine deutlich höhere Stabilität auszeichnen.

Zur vorliegenden Erfindung gehört auch die Herstellung der therapeutischen Mittel oder pharmazeutische Zubereitungen, die mit üblichen Trägerstoffen oder Verdünnungsmitteln und gegebenenfalls üblichen pharmazeutisch-technischen Hilfsstoffen, entsprechend der gewünschten Applikationsart mit einer zur Anwendung geeigneten Dosierung, in an sich üblicher Weise insbesondere durch Vermischen erfolgt.

Die therapeutischen Mittel enthalten die erfindungsgemäß zu verwendenden Verbindungen bei lokaler Anwendung in 0,001 bis 1,0 %iger Konzentration, bevorzugt in 0,01 bis 0,1 %iger Konzentration, und bei systemischer Anwendung vorzugsweise in einer Einzeldosis von 0,1 bis 5 mg.

Dabei kommen als Tagesdosis 50 bis 100 mg in Betracht, wobei die Dosierungen je nach Art und Schwere der Erkrankung, der Zubereitung und der Applikationsart variieren können.

Es werden die üblichen galenischen Zubereitungen verwendet, für die orale Applikation beispielsweise Tabletten, Filmtabletten, Dragees, Kapseln, Pillen, Pulver, Granulate, Lösungen oder Suspensionen. Für die äußerliche Anwendung kommen insbesondere Pasten, Salben, Gele, Cremes, Lotionen, Puder, Lösungen oder Emulsionen und Sprays in Betracht.

Die erfindungsgemäßen Mittel gelangen insbesondere zur innerlichen und äußerlichen Anwendung. Sie werden vorzugsweise oral oder lokal appliziert.

Üblicherweise verwendete pharmazeutisch-technische Hilfsstoffe sind beispielsweise für die lokale Anwendung Alkohole, wie Isopropanol, oxäthyliertes Ricinusöl oder oxäthyliertes hydriertes Ricinusöl, Polyacrylsäure, Glycerinmonostearat, Paraffinöl, (R) Vaseline, Wollfett, Polyäthylenglykol 400, Polyäthylenglykol 400-Stearat sowie äthoxylierter Fettalkohol, für die systemische Anwendung Milchzucker, Saccharose, Propylenglykol und Äthanol, Stärke, Talkum, Polyvinylpyrrolidon.

Weitere übliche Zusätze sind beispielsweise Konservierungsmittel, Antioxidantien, geschmackverbessernde Zusätze, Stabilisierungsmittel, Emulgiermittel, Gleitmittel, Netzmittel usw. Voraussetzung ist, daß alle bei der Herstellung pharmazeutischer Zubereitungen verwendeten Stoffe untoxisch und mit den verwendeten Wirkstoffen verträglich sind (vgl. L. G. Goodman, A. Gilman, The Pharmacological Basis of Therapeutics).

## Beispiel 1

7,8-Dehydro-β-ionylidenethyl-triphenylphosphonium-bromid : In eine Lösung von 66,4 Gewichtsteilen 7,8-Dehydro-β-ionylidenacetaldehyd in 400 Raumteilen Methanol trägt man unter Eisbadkühlung portionsweise 11,6 Gewichtsteile Natriumboranat ein, rührt noch 2 h nach und läßt über nacht bei ca. 20 °C stehen. Man setzt 1 000 Raumteile n-Hexan zu, wäscht mit 1 000 Raumteilen 5-proz. Natriumhydrogentartratlösung und trennt die Phasen. Die wäßrige Phase wird noch dreimal mit je 150 Raumteilen n-Hexan extrahiert. Nach üblicher Aufarbeitung fallen 63,5 Gewichtsteile 7,8-Dehydro-β-ionylidenethanol als farbloses Öl an.

Das 7,8-Dehydro-β-ionylidenethanol (63,5 Gewichtsteile) wird zusammen mit 6,5 Gewichtsteilen Pyridin in 150 Raumteilen Ether gelöst und tropfenweise bei – 20 °C mit einer Lösung von 31,1 Gewichtsteilen Phosphortribromid in 80 Raumteilen Ether versetzt. Man rührt noch 2 h bei ca. 22 °C nach, setzt 300 Gewichtsteile Eis zu und trennt in üblicher Weise zwischen Ether und Wasser. Nach dem Einengen der Etherlösung am Rotationsverdampfer erhält man 75,2 Gewichtsteile 7,8-Dehydro-β-ionylidenethylbromid als hellgelbes Öl.

Zu einer Lösung von 75 Gewichtsteilen 7,8-Dehydro-β-ionyliden-ethylbromid in 150 Raumteilen Methylenchlorid gibt man bei ca. 25 °C tropfenweise eine Lösung von 70,1 Gewichtsteilen Triphenylphosphin in 150 Raumteilen Methylenchlorid, rührt noch 2 h und engt dann bei 50 °C am Rotationsverdampfer ein. Der Rückstand wird in 120 Raumteilen Methylenchlorid aufgenommen, bis zur Trübung mit Ether versetzt, angeimpft und 16 h bei – 8 °C aufbewahrt. Nach dem Absaugen des Kristallisats und Trocknen im Stickstoffstrom erhält man 67 Gewichtsteile 7,8-Dehydro-β-ionyliden-ethyl-triphenylphosphonium-bromid. Weitere 25 Gewichtsteile Kristallisat können nach Zugabe von mehr Ether aus der Mutterlauge isoliert werden, Fp. 166,5° (Zers.).

### Beispiel 2

all-E-7,8-Dehydro-retinsäure :

38,1 Gewichtsteile 7,8-Dehydro-β-ionylidenethyl-triphenylphosphonium-bromid und 11,7 Gewichtsteile E-3-Formyl-2-butensäure-ethylester werden, gelöst in 140 Raumteilen Ethanol, unter Eisbadkühlung tropfenweise mit einer Lösung von 2,56 Gewichtsteilen Natrium in 75 Raumteilen Ethanol versetzt. Man rührt 2 h bei ca. 12 °C, engt dann im Rotationsverdampfer auf ca. 25 % des Volumens ein, setzt 250 Raumteile n-Hexan zu, läßt absitzen und dekantiert die klare Lösung ab, diese operation wird wiederholt. Die Hexanlösung wird dreimal mit je 70 Raumteilen 60 proz. wäßrigem Methanol gewaschen und eingeent : Die Ausbeute beträgt 17,8 Gewichtsteile all-E-7,8-Dehydroretinsäure-ethylester, rotes Öl.

Zur Lösung von 17,5 Gewichtsteilen all-E-7,8-Dehydroretinsäure-ethylester in 150 Raumteilen Ethanol gibt man eine Lösung von 7,1 Gewichtsteilen 85 proz. Kaliumhydroxid in 50 Raumteilen Ethanol und kocht 2 h unter Rückfluß. Diese Lösung wird abgekühlt, mit 100 Raumteilen Wasser versetzt und mit ca. 10 Raumteilen conc. Salzsäure auf pH 2 gestellt. Man extrahiert die wäßrig-ethanolische Phase einmal mit 100 Raumteilen und dreimal mit je 50 Raumteilen Chloroform, wäscht die Chloroformphase dreimal mit je 50 Raumteilen Wasser und engt ein. Die Ausbeute beträgt 18,8 Gewichtsteile rohe all-E-7,8-Dehydro-retinsäure. Das Rohprodukt wird bei – 20 °C aus Diisopropylether kristallisiert. Man erhält 6,6 Gewichtsteilen vom Fp. 106,6 °C. Die Mutterlauge wird eingeengt, der Rückstand in wenig n-Hexan aufgenommen und die Lösung mit Impfkristallen versetzt. Nach ca. 26 h bei 0 °C sind weitere 4,6 Gewichtsteile kristallines Produkt angefallen ; Fp. 158,8 °C. Wie die HPLC-Analyse zeigt, ist das Produkt frei von cis-Isomeren.

### Beispiel 3

13-Z-7,8-Dehydro-retinsäure :

In eine Suspension von 652 Gewichtsteilen 7,8-Dehydro-ß-ionylidenethyl-triphenyl-phosphonium-bromid in 3 000 Raumteilen trockenem Dioxan läßt man bei 10 °C 241 Gewichtsteile n-Butyllithium gelöst in 1 370 Raumteilen n-Hexan einlaufen und rührt 30 min nach. Dann gibt man bei 10 °C eine Lösung von 150 Gewichtsteilen 2-Hydroxy-3-methyl-dihydrofuran-2-on zu und rührt noch 16 h.

Zur Aufarbeitung wird vorsichtig mit 2 000 Raumteilen Wasser und 1 000 Raumteilen n-Hexan versetzt, getrennt und die wäßrige Phase nochmals mit 2 000 Raumteilen n-Hexan extrahiert. Die wäßrige Phase säuert man mit 1 molarer Schwefelsäure auf pH 2 an, extrahiert dreimal mit je 2 000 Raumteilen n-Hexan und wäscht die Hexanlösung zweimal mit je 2 000 Raumteilen 60 proz. wäßrigem Methanol. Die Hexanphase wird bei 50 °C auf 1 500 Raumteile konzentriert. Das bei 22 °C innerhalb von 2 h auskristallisierende Produkt wird durch Filtration isoliert, mit 200 Raumteilen n-Hexan von – 30 °C gewaschen und im Stickstoffstrom getrocknet : 127,5 Gewichtsteile 13-Z-7,8-Dehydro-retinsäure, Fp. 168-170°, $E_1^1$(iso-Propanol) 1 537, 1 545. Die Struktur wurde H-NMR-spektroskopisch sichergestellt. Nach HPCL-Analyse liegt die Isomereneinheit bei 97 %.

Aus der Mutterlauge erhält man nach 16 h bei 20 °C weitere 22 Gewichtsteile kristalline 13-Z-7,8-Dehydro-retinsäure, Fp. 167 °C.

Pharmazeutische Zubereitungen oder Arzneistoffträger für die äußere Anwendung sind z.B. :

### Beispiel 1

Lösung

| | |
|---|---|
| all-E-7,8-Dehydroretinsäure | 0,25 g |
| oxäthyliertes hydriertes Ricinusöl | 35,0 g |
| (R)(Cremophor RH 40, Hersteller BASF AG, Ludwigshafen) | |
| Polyäthylenglykol 400 | 35,0 g |
| oxäthyliertes Ricinusöl (R)(Softigen 767, | |

| | | |
|---|---|---|
| Hersteller Chemische Werke, Witten) | | 10,0 g |
| demineralisiertes Wasser | ad | 100,0 g |

Cremophor RH 40 und Softigen 767 werden gemischt und auf 70 °C erhitzt. Die Wirksubstanz wird unter Rühren gelöst und Polyäthylenglykol 400 zugesetzt. Die Lösung wird dann auf 40 °C abgekült und unter Rühren wird langsam auf 40 °C erhitztes Wasser zugesetzt. Die fertige Lösung wird filtriert und in z.B. 100 ml Flaschen abgefüllt.

Beispiel 2

Creme

| | | |
|---|---|---|
| 13-Z-7,8-Dehydroretinsäure | | 0,1 g |
| Butylhydroxytoluol | | 0,1 g |
| Glycerinmonostearat | | 11,0 g |
| Polyäthylenglykol 400-Stearat | | 6,0 g |
| äthoxylierter Fettalkohol | | 4,0 g |
| Paraffinöl | | 10,0 g |
| p-Hydroxybenzoesäureester (R)(Nipasteril, Hersteller Nipalaboratorium Hamburg) | | 0,2 g |
| Parfumöl | | 0,1 g |
| demineralisiertes Wasser | ad | 100,0 g |

Die Fette werden geschmolzen und die feinstgepulverte Wirksubstanz sowie Butylhydroxytoluol unter Rühren bei 65 °C darin verteilt (Lösung I). Das Wasser wird mit dem Nipaester aufgekocht und auf 65 °C abgekült (Lösung II). In kleinen Anteilen wird Lösung II unter gutem Rühren in Lösung I einemulgiert. Nach dem Abkühlen auf 45 °C wird das Parfumöl zugesetzt und die Emulsion unter Rühren auf Zimmertemperatur abgekühlt. Die fertige Creme wird in innenschutzlackierte Tuben abgefüllt.

Beispiel 3

Gel

| | | |
|---|---|---|
| all-E-7,8-Dehydroretinsäure | | 0,01 g |
| Butylhydroxytoluol | | 0,1 g |
| oxäthyliertes Ricinusöl (Cremophor EL, Hersteller BASF AG, Ludwigshafen) | | 35,0 g |
| Isopropanol | | 20,0 g |
| Polyacrylsäure (R)(Carbopol, Hersteller Goodrich Hamburg) | | 1,5 g |
| Triäthanolamin | | 0,002 g |
| p-Hydroxybenzoesäureester (Nipasteril, Hersteller Nipalaboratorium Hamburg) | | 0,2 g |
| demineralisiertes Wasser | ad | 100,0 g |

Das Cremophor EL wird auf 60 °C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in dem Wasser verteilt (Lösung II). Lösung II wird unter gutem Rühren in kleinen Anteilen zu Lösung I gemischt. Der pH-Wert des Gemisches wird mit Triäthanolamin auf 4,5 eingestellt. Das fertige Gel wird in innenschutzlackierte Tuben abgefüllt.

Für die systemische Anwendung besonders geeignete Zubereitungen oder Arzneistoffträger sind z.B.:

Beispiel 4

Tropfen

| | | |
|---|---|---|
| all-E-7,8-Dehydroretinsäure | | 0,1 g |
| Propylenglykol | | 25,0 g |
| Äthylalkohol | ad | 50,0 g |

Äthylalkohol und Propylenalkohol werden miteinander gemischt und die Wirksubstanz unter Erwärmen auf 35 °C und unter Rühren gelöst. Nach Filtration wird die Lösung in dunkle Tropfflaschen abgefüllt.

Beispiel 5

Hartgelatinekapseln

| | | |
|---|---|---|
| 13-Z-7,8-Dehydroretinsäure | | 1 mg |
| Milchzucker | ad | 0,25 g |

Die Bestandteile werden gesiebt, gemischt und auf einer geeigneten Kapselfüll- und -verschlußmaschine in Hartgelatinekapseln der Größe 2 gefüllt.

**Ansprüche**

1. Pharmazeutisches Mittel, gekennzeichnet durch einen Gehalt an all-E- oder 13-Z-7,8-Dehydroretinsäure als Wirkstoff in 0,001 bis 1,0 %iger Konzentration bei lokaler und in Einzeldosen von 0,1 bis 5 mg bei systemischer Anwendung neben üblichen Trägerstoffen oder Verdünnungsmitteln und pharmazeutisch-technischen Hilfsstoffen.

2. All-E- oder 13-Z-7,8-Dehydroretinsäure zur Anwendung bei der topischen und systemischen Therapie und Prophylaxe von Praekanzerosen und Karzinomen der Haut, Schleimhäute und innerer Organe und bei dermatologischen Erkrankungen.

3. Verfahren zur Herstellung eines pharmazeutischen Mittels, nach Anspruch 1, dadurch gekennzeichnet, daß man all-E- oder 13-Z-7,8-Dehydroretinsäure mit üblichen pharmazeutischen Trägerstoffen oder Verdünnungsmitteln und pharmazeutisch-technischen Hilfsstoffen in an sich üblichen Weise miteinander vermischt.

**Claims**

1. A pharmaceutical agent characterized by a content of all-E- or 13-Z-7,8-dehydroretinic acid as the active compound in a concentration of from 0.001 to 1 % for local application and in a single dose of from 0.1 to 5 mg for systemic administration, in addition to conventional carriers or diluents, and pharmaceutical excipients.

2. All-E- or 13-Z-7,8-dehydroretinic acid for use in the topical and systemic therapy and prophylaxis of pre-cancerous conditions and carcinomas of the skin, mucous membranes and internal organs, and of dermatological disorders.

3. A process for the preparation of a pharmaceutical agent as claimed in claim 1, characterized in that all-E- or 13-Z-7,8-dehydroretinic acid is mixed in the conventional manner with conventional pharmaceutical carriers or diluents, and pharmaceutical excipients.

**Revendications**

1. Agent pharmaceutique, caractérisé par une teneur en acide all-E- ou 13-Z-7,8 déhydrorétinoïque comme principe actif, en concentration de 0,001 à 1,0 % pour application locale et en doses individuelles de 0,1 à 5 mg pour application systémique, à côté de véhicules et diluants usuels et d'auxiliaires de la technique pharmaceutique.

2. Acide all-E- ou 13-Z-7,8 déhydrorétinoïque pour application en thérapie topique et systémique et prophylaxie d'atteintes précancéreuses et de carcinoines de la peau, des muqueuses et organes internes et en dermatologie.

3. Procédé de préparation d'un agent pharmaceutique selon la revendication 1, caractérisé par le fait qu'on mélange entre eux, de manière usuelle, de l'acide all-E- ou 13-Z-7,8 déhydrorétinoïque avec des véhicules ou diluants pharmaceutiques usuels et des auxiliaires de la technique pharmaceutique.